# EUROPEAN PATENT SPECIFICATION

(11) **EP 2 218 450 B1**
(45) Date of publication and mention of the grant of the patent: **05.04.2017**
(21) Application number: 10151616.9
(22) Date of filing: 26.01.2010
(51) Int. Cl.: A61K 31/13, A61P 25/18, A61P 25/24

(54) **Memantine for treating bipolar mood disorders resistant to conventional treatments**
Memantine zur Behandlung der konventionellen Behandlungsresistenten bipolaren affektiven Störungen
Memantine pour le traitement des troubles de l'humeur bipolaires resistants aux traitements conventionnels

(30) Priority: 11.02.2009 IT MI20090174
(43) Date of publication of application: 18.08.2010
(73) Proprietor: Serra, Gino, 09100 Cagliari (IT)
(72) Inventor: Serra, Gino, 09100 Cagliari (IT)
(74) Representative: Minoja, Fabrizio

(56) References cited:
- CHEI TUNG TENG AND FREDERICO NAVAS DEMETRIO: "Memantine may acutely improve cognition and have a mood stabilizing effect in treatment-resistant bipolar disorder [2]" REVISTA BRASILEIRA DE PSIQUIATRIA 2006 BR, vol. 28, no. 3, 2006, pages 252-254, XP002546403
- ZDANYS KRISTINA ET AL: "A systematic review of off-label uses of memantine for psychiatric disorders" PROGRESS IN NEURO-PSYCHOPHARMACOLOGY & BIOLOGICAL PSYCHIATRY, vol. 32, no. 6, August 2008 (2008-08), pages 1362-1374, XP002546404 ISSN: 0278-5846
- ATHANASIOS KOUKOPOULOS ET AL: "Antimanic and mood-stabilizing effect of memantine as an augmenting agent in treatment-resistant bipolar disorder", BIPOLAR DISORDERS, vol. 12, no. 3, 1 May 2010 (2010-05-01), pages 348-349, XP055290705, DK ISSN: 1398-5647, DOI: 10.1111/j.1399-5618.2010.00803.x
- Emea: "The European Agency for the Evaluation of Medicinal Products Evaluation of Medicines for Human Use COMMITTEE FOR PROPRIETARY MEDICINAL PRODUCTS (CPMP) NOTE FOR GUIDANCE ON CLINICAL INVESTIGATION OF MEDICINAL PRODUCTS FOR THE TREATMENT AND PREVENTION OF BIPOLAR DISORDER DISCUSSION IN THE EFFICACY WOR", , 26 April 2011 (2011-04-26), XP055290720, Retrieved from the Internet: URL:http://www.ema.europa.eu/docs/en_GB/do cument_library/Scientific_guideline/2009/0 9/WC500003528.pdf [retrieved on 2016-07-22]
- AGARWAL V. AND TRIPATHI A.: "Memantine in the management of a clinically challenging case of bipolar disorder", INDIAN JOURNAL OF PSYCHIATRY, vol. 51, no. 2, April 2009 (2009-04), - June 2009 (2009-06), pages 137-138, XP008180912,
- KECK PAUL E JR ET AL: "Memantine efficacy and safety in patients with acute mania associated with bipolar I disorder: a pilot evaluation.", CLINICAL NEUROPHARMACOLOGY 2009 JUL-AUG, vol. 32, no. 4, July 2009 (2009-07), pages 199-204, XP008180911, ISSN: 1537-162X

## Description

The present invention refers to the use of Memantine for the preparation of an antimanic and mood stabilizer medication to treat treatment-resistant bipolar mood disorders.

### Background of the invention

Memantine (trade name Ebixa®) is a drug currently used to treat moderate-to-severe Alzheimer's disease. Memantine is a non-competitive selective inhibitor of the NMDA glutamate receptor (1). It has a low affinity for the receptor compared to more powerful NMDA antagonists such as phencyclidine, ketamine and MK-801 (1).

In animal models, Memantine is able to improve learning and memory deficits and reduce neurodegenerative effects (in various cerebral areas implicated in the processes of learning and memory) caused by injuries of various kinds including the administration of NDMA agonists (2,3,4).

The clinical effectiveness of Memantine in moderate-to-severe Alzheimer's disease has been shown by several studies (5) and has been subsequently confirmed in post-marketing studies (6), although its actual efficacy on the patient's quality of life has proven to be moderate and not superior to cholinesterase inhibitors (Donezepil, Rivastigmine, Galantamine) (7), drugs used before Memantine was marketed.

In conclusion, today's clinical experience warrants recognition of Memantine as equally effective (albeit of modest impact if one considers its overall effect on the patient's quality of life) as cholinesterase inhibitors (6,7) and well tolerated in elderly patients like Alzheimer's sufferers (8).

On the basis of recent hypothesis concerning an involvement of the glutamatergic system in schizophrenia, depression, anxiety disorders, obsessive compulsive disorder, etc. (9). Memantine has been used off-label in several psychiatric disorders. The literature reports a number of controlled clinical trials and open case reports in which Memantine was used in a number of psychiatric disorders.

### Major Depression

After observing an antidepressant-like effect of NMDA blockers in some animal models (10,11) and above all in view of the so-called "neurotrophic hypothesis" of depression (presuming that the neurobiological background of depression resides not only in functional but above all in structural damage, with a loss of neurons) (12), many authors think that NDMA glutamate receptor blockers, by antagonizing the excitotoxic action of glutamate, may have a neuroprotective (13) and hence potentially antidepressant action. For this reason Memantine, the only NMDA blocker currently in clinical use, has been the subject of several clinical trials aimed to show its antidepressant effect (14-22). The results have been contradictory and, ultimately, they did not confirm any possible antidepressant action thereof (9).

Two cases of treatment of bipolar depression are allegedly reported (23). A 29-year-old woman with a presumed diagnosis of BD II, suffering for two years from a severe depression resistant to various antidepressant treatments, was treated with 20 mg/die of Memantine in combination with conventional antidepressant treatment. Within a week, the patient showed an improvement in various depressive symptoms. The other case concerned a 32-year-old male patient allegedly diagnosed as BD I, suffering from a mixed state with prevalently depressive symptoms. In this case, too, Memantine (10 mg/die) was administered with an improvement in the depressive symptoms.

The two patients however cannot properly be considered as affected by bipolar disorders and the observed effect after Memantine administration cannot be defined as mood stabilizing, being rather a modest acute antidepressant effect. The title of the report is misleading.

In fact, the first case, mistakenly diagnosed BD Type II, no episode of hypomania, an essential requisite for a correct diagnosis of BD II, was reported. The patient actually showed a history of chronic major depression, with brief and insignificant improvements, associated in certain periods with eating disorders and numerous suicide attempts, treated for many years with antidepressants (continuosly and at very high doses: consistent with the clinical history of a persistent depressive symtomatology) and mood stabilizers with little success. According to the very same statements by the authors, Memantine had a modest antidepressant effect for a week (or perhaps for a month) but certainly not a mood-stabilizing effect, which by definition is a long-term effect (mood-stabilizing effect actually refers to prevention of depressive and/or manic/hypomanic recurrences in the long term). The diagnosis was therefore incorrect, being rather a case of chronic (or recurrent) Major Depression and not BD Type II.

Also the other case has been incorrectly diagnosed as BD Type I (in fact the table shows that the first episode, "unipolar depression with OCD symptoms", follows a supposed short episode of "full mixed state" followed by a melancholic depression from 02/02 to 03/02"). After this episode, the clinical history was marked by a chronic depression treated with antidepressants (continuosly and at very high doses: consistent with the clinical history of a persistent depressive symtomatology) and mood stabilizers with little results: there is no evidence of a manic or mixed episode from 03/02 until the index episode which the authors erroneously diagnose as a mixed state: it is therefore a history incompatible with BD I.

The diagnosis of "Mixed state mostly with depressive symptoms" is wrong. The DSM-IV criteria for Mixed states require that "in the same episode there should be present symptoms of Major Depression and Manic Episode". Here the authors observe hypomanic episodes or "hypomanic outbursts" of compulsive shopping, binge eating and lack of insight (actually, hypomanic outbursts do not exist, instead outburts of compulsive behaviour can be observed in OCD). Even admitting that these may be symptoms of hypomania, this would not be enough to justify a diagnosis of a mixed state. In fact, they are OCD (obssesive compulsive disorder) symptoms, from which the patient suffered from the outset. Therefore, the correct diagnosis is Major Depression with OCD symptoms. The effect of Memantine is a "rapid improvement in depressive symptoms. Despite continuing impulsiveness, his insight into compulsive shopping behaviour and binge eating improved". Therefore, Memantine had a modest antidepressant effect - not mood-stabilizing - and slightly attenuated the OCD symptoms (an effect consistent with several recent observations that Memantine improves OCD). In this case, too, the report did not referred to a BPI patient, in a mixed state and the observed effect of Memantine was simply antidepressant (and anti-OCD) and not mood stabilizing (which by definition is a long-term effect, while here an acute effect is described).

Indeed, the authors themselves conclude that "The patients described in this report used medications that could cause cognitive impairment. Memantine could have antagonized these effects, through unclear mechanisms. To our knowledge, this is the first report on the usefulness of Memantine in the treatment of cognitive impairment in bipolar disorder patients" and do not stress the hypothetical mood-stabilizing effect.

### Bipolar disorders

An animal model of mania is reported by Gao Yonglin et al., in Biological Psychiatry, vol. 63, no. 7, Suppl. S, April 2008 (2008-04), page 57S, 63RD Annual Convention of the Society-of-Biological-Psychiatry; Washington, dc, USA; 2008. This model has not found extensive application and it suggests at most an antimanic effect of Memantine similar to that of lithium, certainly not an antimanic effect in lithium-resistant disorders.

Kavirajan H. Expert Opin Drug Saf. 2009 Jan;8(1):89-109. Review. suggested the use of Memantine in the treatment of BPD, both as an antimanic agent and mood stabilizer.

Bipolar disorders (with any pharmacological rationale or preliminary clinical observation) are mentioned in WO 2006/034465, US2005/203191 and in WO2005/000216.

### Schizophrenia

Three case reports have shown an improvement in the symptoms in patients affected by catatonic schizophrenia (24-26). Memantine was administered at a dosage of 10 mg twice a day in two patients and 10 mg per day in the other patient. No improvement was observed, however, in a recent controlled clinical trial, in which Memantine was administered in combination with an atypical antipsychotic (27).

### Obsessive Compulsive Disorder

Two case reports describing the effect of Memantine in three patients with OCD have been published (28, 29). Two of the three patients showed symptomatic improvements with Memantine at a dosage of 5-15 mg a day. An open-label study was recently published in which a symptomatic improvement was observed in 6 patients out of 14 after the administration of Memantine (20 mg) in combination with SSRIs(30).

### Pervasive development disorders

Five open clinical studies (31-35) and one case report (36) have shown a symptomatic improvement after the administration of Memantine.

### Eating Disorders

Only one study (37) reported diminished appetite, the disappearance of binge eating (in two out of five patients) and a reduction of body weight in five obese patients affected by binge eating disorder.

### Anxiety disorders

Although there is some clinical and preclinical evidence for the role of the NMDA receptor in the pathogenesis of anxiety disorders (38), no clinical study has assessed Memantine in the treatment of these disorders.

### Addiction

Nine controlled clinical trials and one open-label trial have described the use of Memantine in a number of addictions. Two studies (39, 40) have demonstrated a reduction in craving for alcohol and one (41) has shown a reduction of the symptoms of alcohol withdrawal syndrome. Two studies (42, 43) have shown a reduction in alcohol consumption, but in one of these the effect of Memantine did not significantly differ from placebo. Further, Memantine seems to be effective in reducing the symptoms of heroin withdrawal (44,45), while no benefit has been detected in nicotine (46) or cocaine (47, 48) addiction.

A number of specific therapeutic indications have recently been described in the patent literature, and in particular: Major Depression, Bipolar Disorder for Bipolar Depression, Anxiety Disorders, Drug Addiction (49); NMDA receptor antagonists (including Memantine) in association with SSRI antidepressants in the treatment of depression and other mood disorders (50); child behaviour disorders included in the 'autistic spectrum' and attention-deficit hyperactivity disorder (51); control and prevention of various disorders including acute and chronic neurological disorders (52).

Mood disorders are currently classified (DSM IV TR) (53) as Depressive Disorders and Bipolar Disorders. Depressive Disorders include Major Depressive Disorder (single or recurrent) and Dysthymic Disorder. Bipolar Disorders comprise: BD I (which presents with an alternation of episodes of major depression and recurrent episodes of mania); BD II (which is made up of episodes of major depression and recurrent hypomanias); Cyclothymic Disorder (for at least two years several hypomanic and depressive episodes which must not be major). Further, a Mixed Episode is when symptoms of major depression and mania are present in the same episode. These disorders may sometimes have a rapid-cycling course, marked by the presence of at least four cycles per year (a cycle equals one episode of depression followed by mania, or vice versa), very often resistant to current treatments.

The depression that manifests in BD is also commonly defined, in the scientific literature, as Bipolar Depression, while that of Major Depressive Disorder is often called Unipolar Depression (or Unipolar Disorder). The treatments currently available for treating and preventing mood disorders are antidepressant drugs, electroconvulsive therapy, lithium, certain anticonvulsants (commonly called mood stabilizers) and antipsychotics (54).

Antidepressants are indicated for the treatment of unipolar and bipolar depression, while electroconvulsive therapy (ECT) is generally reserved for severe depressions which do not respond to drugs. The idea of combining lithium with antidepressants (lithium augmentation) is not universally accepted. The use of antidepressants for the prophylaxis/prevention of recurrent unipolar depression is the subject of much debate and is indeed strongly opposed by many.

Lithium is the drug of choice for the treatment of mania and the prophylaxis (as a mood stabilizer) of manic and/or depressive recurrences of BD and Unipolar Depression. In cases of severe mania with a strong component of psychomotor agitation and/or psychotic symptoms, for the treatment of the acute phase, antipsychotic drugs are generally combined.

Anticonvulsants (carbamazepine, valproic acid, etc.) are used (in mania and/or as mood stabilizers) in combination with lithium when the latter does not produce a satisfactory response or in monotherapy in patients for whom lithium is contraindicated. The effectiveness of these drugs, both as antimanics and mood stabilizers, is however modest, and therefore patients who do not respond adequately to lithium (55, 56) today constitute the real problem of long-term treatment of mood disorders. The use of antipsychotics as mood stabilizers is not feasible both because of their dubious efficacy and their long-term safety and/or tolerability. Traditional antipsychotics, as well as having undesirable effects of a psychiatric type (emotional blunting, etc.), may, if administered for a long time, provoke sometimes debilitating and irreversible neurological damage (57). The so-called atypical antipsychotics, on which the jury is still out on whether they do not cause long-term neurological damage, have the drawback (except for some) of being able to cause what is called "metabolic syndrome" (58) which constitutes a serious cardiovascular risk factor and thus risk of early death (which is aggravated by the fact that BD patients per se have an increased cardiovascular risk). Finally, it should be stressed that most antipsychotics, whether traditional or atypical, may cause, albeit rarely, death by sudden cardiac arrest (59).

Concern has also been raised by a recent alarm (60) over the possibility that anti-epileptics may bring a two-fold increase in suicide risk compared to placebo.

The problem of the therapy and prophylaxis of mood disorders in patients who do not respond to lithium, whose numbers appear to be continually rising, therefore remains unsolved (61, 62). Hence the need for new and more effective antimanic and mood-stabilizing drugs.

Among the other causes that may lead to a disease course resistant to lithium and other stabilizers is the ever more widespread inappropriate use of antidepressants, drugs which Ghaemi have described as "mood destabilizers" (62). These drugs, in fact, in a significant number of cases, cause a rapid switch from depression to mania both in BP and UP patients (63). This switch is not prevented by lithium nor by the other stabilizers which, indeed, appear to favour it (63). The induction of mania by antidepressants is viewed as one of the most significant factors in triggering a rapid-cycling course resistant to all the treatments used up to that point (61, 62, 64).

It has been hypothesised (on the basis of experimental results obtained in animal models) that the switch from depression to mania, and therefore the resultant triggering of the rapid-cycling course, induced by antidepressants, depends on their capacity to provoke a sensitization of dopamine receptors (65-69) and hence increase dopaminergic transmission. There is ample evidence, in fact, suggesting that mania is associated with an increase in dopaminergic transmission, while depression may be associated with a reduced dopaminergic function (70). Neither lithium nor other mood stabilizers are able to prevent the development of sensitization of the dopamine receptors induced by antidepressants (71-73).

The increased activity of the dopaminergic system (mania) induced by antidepressants lasts about a week, and is immediately followed by a reduced function of the system, which is accompanied by depression-like behaviour (74, 75). Antidepressants, in other words, because of their capacity to induce mania, are able to induce in animals (as in humans) behaviour similar to rapid-cycling bipolar disorder.

The prevention of mania, whether induced by antidepressants or spontaneous, is the essential element in the therapy and prophylaxis of bipolar disorders (76, 77). When, in fact, treatments currently in use do not achieve this aim the course of the disorder becomes "malign", or refractory to treatment, as in the rapid-cycling course.

A similar phemonenon, i.e. the sensitization of dopaminergic receptors, may also be involved in mania and in mixed episodes in which the excitatory component is seen as prevalent and the administration of antidepressants worsens the spontaneous clinical picture. It is in fact possible that the phenomenon induced by antidepressant drugs (triggering of mania and the rapid-cycling course) is none other than an amplification of a physiopathological phenomenon underlying bipolar disorder (64).

Other forms of behavioural sensitization of the dopaminergic system induced by drugs or stressful situations may be considered a reproduction of mania both in animals and in humans. The repeated administration of amphetamine in moderate doses causes a state of severe manic excitement with paranoid delusions. The presence of this psychotic symptom for a long time mistakenly led this effect to be considered as 'paranoid schizophernia'; in fact, according to present nosology, this symptom may be considered "a manic episode with psychotic features" (78, 79, 70). The fact that a moderate dose of amphetamine produces, after repeated administrations, manic excitement is due to a phenomenon which has been called 'inverse tolerance', but it is undoubtedly more correct to view it as a phenomenon of sensitization of the dopaminergic system: the repeatedly administered dose becomes increasingly effective until it provokes mania. This phenomenon has also been observed in laboratory animals and may be considered an animal model of mania (70).

Cocaine and dopamine receptor stimulants (79), too, after repeated administration, sensitize the dopaminergic system and produce a mania-like behaviour in animals and mania in humans.

A sensitization of dopaminergic receptors and behavioural effects seen as an animal model of mania have also been observed after REM sleep deprivation which has a fleeting antidepressant effect and induces a hypomania/mania state in humans (69).

The proposal to use antiepileptic drugs in the therapy and prophylaxis of mood disorders is based on the hypothesis that the phenomenon of sensitization may play a significant role in the pathogenesis of mood disorders (80-90). It has been suggested, in fact, that the recurrences of mood disorders might be linked to a physiopathological phenomenon similar to kindling (the administration of repeated subconvulsive electric stimuli, by way of sensitization, become increasingly effective, until they bring on convulsions), used to produce animal models of epilepsy. Just as in kindling a sensitization to (sub-convulsive) electrical stimuli is developed until a convulsion is induced, so in mood disorders stimuli of a stressful nature would become over time increasingly effective in triggering single episodes (in individuals who are presumably genetically predisposed) and hence also recurrences of episodes of mania and/or depression. Similarly, the very same recurring episodes would make the individual increasingly vulnerable to stressful stimuli. Therefore, on the basis of the consideration that the neurobiological phenomenon underlying the recurrences of mood disorders may be similar to that of epilepsy, the use of carbamazepine was proposed as an antimanic and mood stabiliser (80), and indication later extended to valproic acid and eventually to all the new-generation antiepileptics.

Finally, there is ample experimental evidence showing that the NMDA glutamate receptor plays an essential role in the phenomenon of sensitization. Its stimulation is, in fact, necessary for the sensitization of amphetamine (91-96), methylphenidate (97), cocaine (98-101), apomorphine (94, 102, 103) and other dopamine mimetics (104, 105), nicotine (106), morphine (107, 108), ethanol (109-111), as well as several types of stress such as, for instance, "restrain stress" (95) and "social defeat stress" (112).

The stimulation of NMDA receptors is required for the development of dopamine receptor sensitization induced by antidepressants. Indeed, we found that the administration of MK.801, a selective non-competitive NMDA receptor blocker, completely prevents the dopamine receptor sensitization induced by imipramine (113) and by electroconvulsive shock (114). On the contrary the competitive NMDA receptors antagonist CPP fails to pevent the dopamine D2 receptors sensitization induced by imipramine (115).

These observations strongly suggest that the non-competitive, but not the competitive, blockade of NMDA receptors should result in an anti-manic and mood stabilizing action, and that, more important, it should also be effective in the treatment of the disorders resistant to currently used antimanic and mood stabilizers.

### Description of the invention

It has now been found that Memantine is an useful antimanic and mood stabilizer for the treatment of bipolar mood disorders resistant to treatments currently in use, particularly for the treatment of bipolar mood disorders resistant to lithium treatment.

Memantine is particularly useful in the prevention of the switch from depression to mania/hypomania, spontaneous or induced by antidepressant treatments, for the therapy of mania and prophylaxis of type I and II bipolar disorders resistant to currently used treatments, for the therapy and prophylaxis of rapid-cycling bipolar disorder resistant to treatments currently in use, for the therapy and prophylaxis of mixed bipolar episodes resistant to treatments currently in use and for the therapy and prophylaxis of bipolar disorder with psychotic symptoms resistant to standard treatments. It has been found, in particular, that Memantine is the only NMDA blocker proposed as antimanic and mood stabilizer which is able to prevent recurrences of the disorder, whether manic or depressive.

The use described in the present invention is absolutely at variance with prevalent hypotheses which take Memantine to be a potentially antidepressant drugs. The countless attempts to show a possible antidepressant effect have all proven to be contradictory and ultimately negative.

The antimanic and mood stabilizing effect of Memantine according to the invention has been demonstrated by preliminary clinical results. The clinical trials were conducted on patients who have been for many years resistant to all possible treatments hitherto available. The fact that Memantine was administered in addition to the current therapy (for obvious ethical reasons) does not affect the value of the observation, since the current therapies were ineffective, just as all the other therapeutic strategies adopted before the current therapy had proven to be ineffective. We report, in order to exemplify, the most clinically significant cases in bipolar patients (DSM IV-TR) resistant to hitherto available treatments.

From the preliminary clinical data it appears evident that Memantine exerts a meaningful antimanic and mood-stabilizing action in patients not responding to standard treatments.

Memantine was administered at 10-30 mg/day (according to the doses used for Alzheimer Disease and in many off-label uses of the drug in other psychiatric disorders) to 18 bipolar patients (mean age 42, range 24-61, SD 11): 13 BP type 1 (11 women and 2 men) and 5 BP type 2 (3 women and 2 men). Ten of these 18 patients were rapid cyclers, 5 were continuous circular with long cycles and 3 had a course with free intervals. Thirteen exhibited psychotic symptoms. These patients had been ill for an average of 21.5 years (range 5-47, SD 12) and they had been resistant to very intense standard treatments, including lithium, anti-epileptics, typical and atypical antipsychotics, ECT and antidepressants. The 10 rapid cyclers had a mean duration of RC course of 11 years (range 3-29, SD 7,6).

The Memantine treatment was added to the ongoing treatment which was left unmodified. The severity of the patients' condition before Memantine, and the change after Memantine addition was evaluated on the CGI-BP Overall Bipolar Illness scale:
CGI-BP Overall Bipolar Illness: Severity
   1. Normal, not ill;
   2. Minimally ill;
   3. Mildly ill;
   4. Moderately ill;
   5. Markedly ill;
   6. Severely ill;
   7. Very severely ill.

All patients have been followed-up for 24 weeks. The average of CGI-BP score was 6.6 (range 5-7) before the addition of Memantine. CGI-BP overall Bipolar Illness: Change from preceding phase
1. Very much improved
2. Much improved
3. Minimally improved
4. No change
5. Minimally worse
6. Much worse
7. Very much worse

Compared to the preceding phases of illness, at 24 weeks, 9 patients scored 1 (very much improved), 4 patients scored 2 (much improved), 2 patients scored 3 (minimally improved) and 3 patients remained unchanged (score 4). Among the ten rapid cyclers, six reached stability. Thus after 24 weeks of Memantine treatment 72.2% of patients were very much or much improved. The mean time to improvement was 55 days (range 6-132, SD 48,4). These results suggest a meaningful anti-manic and mood-stabilizing effect of Memantine. The improvement was obtained in patients who had been resistant for many years to all available treatments and at relatively small doses. Side-effects: one patient complained of dizziness and another of constipation.

Memantine may be administered in dosages similar to those in normal clinical use, using the conventional ways of administration, in particular orally in the form of tablets, capsules or drops. The effective dosages range from about 10 to about 30 mg per day but they may be varied by the doctor according to the observed clinical response. The duration of treatment may last up to the complete improvement of manic symptoms and maintenance therapy may continue in order to prevent manic and/or depressive relapses, until the disorder is completely stabilized.

According to the algorithm recently proposed by Motola et al (116), Memantine possesses all the characteristics to be considered an important therapeutic innovation. In fact, the drug is effective in treatment-resistant bipolar disorder (i.e. in patients who have no therapeutic alternatives), a severe and disabling illness. Moreover, its safety and good tolerability is well documented by more than 20 years of clinical use.

### BIBLIOGRAPHY

1) Johnson JW et al., Curr Opin Pharmacol. 2006 Feb;6(1):61-7.
2) Nakamura S et al., Eur J Pharmacol. 2006 Oct 24;548(1-3): 115-22.
3) Wenk GL et al., Behav Pharmacol. 2006 Sep; 17(5-6): 411-24.
4) Keilhoff G et al., Eur J Pharmacol. 1992 Sep 4; 219(3): 451-4.
5) EPAR (European Public Assessment Report) on Exiba; EMEA 15 May 2002.
6) Emre M et al., J Alzheimers Dis. 2008 Jun;14(2):193-9.
7) Kaduszkiewicz H et al., Evid Based Ment Health. 2008 Nov; 11(4): 113.
8) Farlow MR et al., Drug Saf. 2008; 31(7): 577-85.
9) Zdanys K et al., Prog Neuropsychopharmacol Biol Psychiatry. 2008 Aug 1; 32(6): 1362-74.
10) Rogóz Z et al., Neuropharmacology. 2002 Jun;42(8):1024-30.
11) Trullas R et al., Eur J Pharmacol, 1990 Aug 21; 185 (1):1-10.
12) Schmidt HD et al., Behav Pharmacol 2007; 18(5-6): 391-418.
13) Volbracht C et al., Eur J Neurosci. 2006 May;23(10):2611-22.
14) Zarate CA et al., Am J Psychiatry. 2006 Jan; 163(1):153-5.
15) Kollmar R et al., Aust N Z J Psychiatry. 2008 Feb; 42(2):170.
16) Ferguson JM et al., Clin Neuropharmacol. 2007 May-Jun; 30(3):136-44.
17) Muhonen LH et al., J Clin Psychiatry. 2008 Mar; 69(3): 392-9.
18) Munoz C et al., J Neuropsychiatry Clin Neurosci. 2008; 20(1): 119-20.
19) Maeng S, Curr Psychiatry Rep. 2007 Dec; 9(6):467-74.
20) Tsai GE, Arch Gen Psychiatry. 2007 Sep; 64(9): 1099-100; author reply 1100-1.
21) Pittenger C et al., CNS Neurol Disord Drug Targets. 2007 Apr; 6(2):101-15.
22) Zarate CA et al., Arch Gen Psychiatry. 2006 Aug;63(8):856-64.
23) Teng CT et al., Rev Bras Psiquiatr. 2006 Sep; 28(3): 252-4.
24) Carroll B et al., Ann Clin Psychiatry. 2006 Apr-Jun; 18(2): 133-4.
25) Carpenter S et al., Ann Pharmacother. 2006 Feb; 40(2): 344-6.
26) Thomas C et al., Am J Psychiatry. 2005 May; 162(5):1042.
27) Lieberman JA, Neuropsychopharmacology, 2008 Nov 12.
28) Poyurovsky M et al., Am J Psychiatry.2005 Nov;162(11):2191-2.
29) Pasquini M et al., Prog Neuropsychopharmacol Biol Psychiatry. 2006 Aug 30;30(6):1173-5.
30) Aboujaoude E et al., J Clin Psychopharmacol 2009 Feb; 29 (1): 51-5.
31) Owley T et al., J Child Adolesc Psychopharmacol 2006;16(5): 517-24.
32) Chez MG et al., J Child Neurol 2007;22(5):574-9.
33) Erickson CA et al., Psychopharmacology 2007;191:141-7.
34) Findling RL et al., J Child Adolesc Psychopharmacol 2007; 17(1):19-33.
35) Niederhofer H, J Clin Psychopharmacol 2007; 27(3): 317.
36) Erickson CA et al., J Clin Psychiatry 2006;67(6):1000.
37) Hermanussen M et al., Econ Hum Biol 2005;3: 329-37.
38) Cortese B et al., CNS Spectrums 2005; 10(10):820-30.
39) Bisaga A et al., Psychopharmacology 2004; 172:16-24.
40) Krupitsky EM et al., Am J Psychiatry 2007a; 164(3):519-23.
41) Krupitsky EM et al., Alcoholism: Clin Exp Res 2007b; 31(4): 604-11.
42) Arias AJ et al., Addic Disord Treat 2007; 6(2):77-83.
43) Evans SM et al., Alcoholism: Clin Exp Res 2007; 31(5):775-82.
44) Bisaga A et al., Psychopharmacology 2001; (157):1-10.
45) Krupitsky EM et al., Addict Disord Treat 2002; 1(4):143-6.
46) Thuerauf N et al. J Neural Transm 2007; 114:351-7.
47) Collins ED et al., Behav Pharmacol 1998; 9(7):587-98.
48) Collins ED et al., Pharmacol Biochem Behav 2006; 83(1):47-55.
49) WO 2002/45710.
50) US 20050014743.
51) WO 2006/034465.
52) US 20070299113.
53) DSM-IV-TR: American Psychiatric Association. 2000.
54) Baldessarini RJ in Goodman & Gilman's The Pharmacological Basis of Therapeutics 11 Edition 2005; 429-500.
55) Koukopoulos A et al., Adv Biochem Psychopharmacol. 1995; 49:127-47.
56) Tohen M et al., Am J Psychiatry. 2005 Jul; 162(7):1281-90.
57) Serra G, Gessa GL Manuale di Psicofarmacologia Masson Editore, 1990.
58) D'Aquila PS et al., Enciclopedia Medica Italiana, III Aggiornamento della Seconda edizione. USES Edizioni Scientifiche. Coll. 2447-2450.
59) Ray WR et al., N Engl J Med 2009. 360: 225-35.
60) Warnings About Suicidality Risk With Antiepileptic Drugs FDA, Alert 2008, Dec 16.
61) Kukopulos A et al., Pharmakopsychiatr. Neuropsychopharmakol. 1980 Jul; 13(4):156-67.
62) Ghaemi SN Am J Psychiatry. 2008 Mar; 165(3):300-2.
63) Tondo L et al., Acta Psychiatr Scand.2009 Dec 2.
64) Tondo L et al., Pharmacopsychiatry. 1981; 16(2):119-23.
65) Serra G et al., Life Sci. 1979 Jul 30; 25(5): 415-23.
66) Serra G et al., Pharmacol Toxicol. 1992; 71 Suppl 1:72-85.
67) Serra G et al., Brain Res. 1990 Sep 17; 527(2):234-43.
68) D'Aquila PS et al., Eur J Pharmacol. 2000 Sep 29;405(1-3):365-73.
69) Gessa GL et al., 1995; Adv Biochem Psychopharmacol. 49:43-66.
70) Fibiger HC Adv Biochem Psychopharmacol. 1995; 49: 1-17.
71) D'Aquila PS et al., Eur J Pharmacol. 2000 Apr 28;3.
72) D'Aquila PS et al., Eur J Pharmacol. 2001 Mar 23; 416(1-2):107-11.
73) D'Aquila PS et al., Eur J Pharmacol. 2006 Mar 27;535(1-3): 208-11.
74) D'Aquila PS et al., Eur J Pharmacol. 2003; Jan 1;458(1-2):129-3.
75) D'Aquila PS et al., Eur J Pharmacol. 2004 May 10;492(1):61-3.
76) Koukopoulos A et al., Int Pharmacopsychiatry 1973, 8 (3): 152-8.
77) Koukopoulos A et al., European Psychiatry 2009, Mar, 24(2): 125-134.
78) Westfall TC, Westfall DP. In Goodman & Gilman's The Pharmacological Basis of Therapeutics 11 Edition 237-295.
79) O'Brien CP. In Goodman & Gilman's The Pharmacological Basis of Therapeutics 11 Edition 607-627.
80) Post RM et al., J Clin Psychiatry, 1989 Dec; 50 Suppl:23-30; discussion 45-7.
81) Post RM, Pharmacopsychiatry, 1990 Jan; 23(1):3-17.
82) Post RM, Pharmacopsychiatry, 1992 Jan; 25(1):41-3.
83) Atre-Vaidya N et al., J Neuropsychiatry Clin Neurosci. 1997 Fall;9(4):525-33.
84) Antelman SM et al., Prog Neuropsychopharmacol Biol Psychiatry. 1998 Jan; 22(1):65-78.
85) Kraus JE, J Neuropsychiatry Clin Neurosci. 2000 Summer; 12(3):328-43.
86) Huber MT et al., J Psychiatr Res. 2001 Jan-Feb; 35(1):49-57.
87) Dienes KA et al., J Affect Disord. 2006 Oct;95(1-3):43-9. 2006 Jul 11.
88) Post RM, Neurosci Biobehav Rev. 2007; 31(6):858-73.
89) Benedetti F et al., Behav Brain Res. 2008 Mar 5;187(2):221-7,2007 Sep 14.
90) Patten SB, Med Hypotheses. 2008 Dec;71(6):872-5. Epub 2008 Sep 2.
91) Wolf ME et al., J Neurosci. 1994 Mar; 14(3 Pt 2):1735-45.
92) Ohmori T et al., Pharmacol Biochem Behav. 1994 Jul; 48(3): 587-91.
93) Vezina P et al., Psychopharmacology. 2000 Aug 151(2-3): 184-91.
94) Battisti JJ et al., Pharmacol Biochem Behav. 2000 Oct;67(2):241-6.
95) Pacchioni AM et al., Ann N Y Acad Sci. 2002 Jun; 965:233-46.
96) Grönig M et al., Naunyn Schmiedebergs Arch Pharmacol. 2004 Feb; 369(2):228-31.
97) Gaytan O et al., Brain Res Bull. 2000; 51(6):485-92.
98) Li Y et al., Psychopharmacology (Berl). 2000 Aug; 151(2-3): 175-83.
99) Heusner CL et al., J Neurosci. 2005 Jul 13; 25(28): 6651-7.
100) Rompré PP et al., Brain Res. 2006 Apr 26;1085(1):77-86, 2006 Mar 30.
101) Kim HS et al., Brain Res Bull. 1996; 40(3): 201-7.
102) Võikar V et al., Eur J Neuropsychopharmacol. 1999 Dec; 9(6): 507-14.
103) Acerbo MJ et al., Behav Brain Res. 2004 May 5; 151(1-2):201-8.
104) Kalivas PW, Drug Alcohol Depend. 1995 Feb; 37(2): 95-100.
105) Rockhold RW, Prog Drug Res. 1998; 50:155-92.
106) Kelsey JE et al., Psychopharmacology (Berl). 2002 Jun; 161(4):370-8.
107) Jeziorski M et al., Synapse. 1994 Feb; 16(2):137-47.
108) Trujillo KA, Neurotox Res. 2002 Jun;4(4): 373-91.
109) Broadbent J et al., Alcohol Alcohol. 1999 May-Jun; 34(3):283-8.
110) Camarini R et al., Alcohol Clin Exp Res. 2000 Mar; 24(3):285-90.
111) Kotlinska J et al.,. Behav Pharmacol. 2006 Feb; 17(1):1-8.
112) Yap JJ et al., Psychopharmacology (Berl). 2005 Apr; 179(1): 230-9.
113) D'Aquila PS et al., Eur. J. Pharmacol. 1992 224: 199-202*.*
114) D'Aquila PS et al., Eur. J. Pharm.1997 330, 11-14*.*
115) D'Aquila PS et al., XII Congresso della Società Italiana di Neuropsicofarmacologia, "La Neuropsicofarmacologia nel terzo millennio: un tributo a Gian Luigi Gessa", 7-10 giugno 2000, Domus De Maria, Cagliari. p. 176.
116) Motola et al Br J Clin Pharmacol. 2005 Apr;59(4):475-478.

## Claims

1. The use of Memantine for the preparation of an antimanic and mood stabilizer medicament for the treatment of bipolar mood disorders resistant to treatments currently in use.

2. The use according to claim 1 wherein the medicament is for the prevention of the switch from depression to mania/hypomania, spontaneous or induced by antidepressant treatments.

3. The use according to claim 1 or 2 wherein the medicament is for the treatment of mania/hypomania and prevention of depressive and manic/hypomanic recurrences in lithium-resistant Type I and II Bipolar Disorder.

4. The use according to claim 1 wherein the medicament is for the treatment of mania/hypomania and the prevention of manic/hypomanic and depressive recurrences in Type I and II Bipolar Disorder when the course is rapid-cycling.

5. The use according to claim 1 wherein the medicament is for the treatment of mania and prevention of manic and depressive recurrences in Type I Bipolar Disorder with psychotic symptoms resistant to standard treatments.

6. The use according to claim 1 wherein the medicament is for the Treatment of mixed episode and prevention of manic/mixed and depressive recurrences of Type I Bipolar Disorder resistant to lithium.

## Patentansprüche

1. Verwendung von Memantin zur Herstellung eines antimanischen und gemütsstabilisierenden Medikaments zur Behandlung von bipolaren Gemütsstörungen, welche resistent gegenüber derzeitig verwendete Behandlungen sind.

2. Verwendung gemäß Anspruch 1, wobei das Medikament zur Prävention von dem Wechsel von Depression zu Manie/Hypomanie, welcher spontan oder durch Antidepressivum-Behandlungen induziert wurde, ist.

3. Verwendung gemäß Anspruch 1 oder 2, wobei das Medikament zur Behandlung von Manie/Hypomanie und Prävention von depressiven und manischen/hypomanischen Rückfällen bei lithiumresistenter Typ-I- und Typ-II-bipolarer Störung ist.

4. Verwendung gemäß Anspruch 1, wobei das Medikament zur Behandlung von Manie/Hypomanie und zur Prävention von manischen/hypomanischen und depressiven Rückfällen in Typ-I- und -II-bipolaren Störungen ist, wenn der Verlauf ein Rapid-Cycling ist.

5. Verwendung gemäß Anspruch 1, wobei das Medikament zur Behandlung von Manie und Prävention von manischen und depressiven Rückfällen bei Typ-I-bipolarer Störung mit psychotischen Symptomen, resistent gegenüber Standardbehandlungen, ist.

6. Verwendung gemäß Anspruch 1, wobei das Medikament zur Behandlung von gemischter Episode und Prävention von manischen/gemischten und depressiven Rückfällen von Typ-Ibipolarer Störung, resistent gegenüber Lithium, ist.

## Revendications

1. Utilisation de mémantine pour la préparation d'un médicament antimaniaque et stabilisateur de l'humeur pour le traitement de troubles affectifs bipolaires résistant à des traitements actuellement en usage.

2. Utilisation selon la revendication 1, dans laquelle le médicament est destiné à la prévention du passage d'une dépression à une manie/hypomanie, spontané ou induit par des traitements antidépresseurs.

3. Utilisation selon la revendication 1 ou 2, dans laquelle le médicament est destiné au traitement d'une manie/hypomanie et à la prévention de récurrences dépressives et maniaques/hypomaniaques dans les troubles bipolaires de type I et II résistant au lithium.

4. Utilisation selon la revendication 1, dans laquelle le médicament est destiné au traitement d'une manie/hypomanie et à la prévention de récurrences dépressives et maniaques/hypomaniaques dans les troubles bipolaires de type I et II lorsque l'évolution est du type à cycles rapides.

5. Utilisation selon la revendication 1, dans laquelle le médicament est destiné au traitement d'une manie et à la prévention de récurrences maniaques et dépressives dans le trouble bipolaire de type I présentant des symptômes psychotiques résistant à des traitements standard.

6. Utilisation selon la revendication 1, dans laquelle le médicament est destiné au traitement d'épisodes mixtes et à la prévention de récurrences maniaques/mixtes et dépressives du trouble bipolaire de type 1 résistant au lithium.
